# EUROPEAN PATENT APPLICATION

(11) **EP 0 863 148 A1**
(43) Date of publication of application: **09.09.1998**
(21) Application number: 97200622.5
(22) Date of filing: 03.03.1997
(51) Int. Cl.: C07H 19/16, A61K 31/70

(54) **Compounds derived from adenosine, use of such compound as a medicament, method for synthesizing such a compound and intermediates thereof**

(71) Applicant: K.U. LEUVEN RESEARCH & DEVELOPMENT, 3000 Leuven (BE); RIJKSUNIVERSITEIT LEIDEN, 2333 CC Leiden (NL)
(72) Inventor: Herdewijn, Piet André Maurits Maria, 311 Rotselaar (BE); Ijzerman, Adriaan Pieter, 2314 EW Leiden (NL)
(74) Representative: Eveleens Maarse, Pieter

(57) **Abstract**

The invention relates to compounds derived from adenosine and analogs thereof, to the use of such compounds in pharmaceutical compositions as a cardiotonic, a renal protectant, a cognition enhancer and/or an antiasthmatic, to a method and to intermediates for synthesizing such a compound.

The compounds according to the invention have the general formula (I): 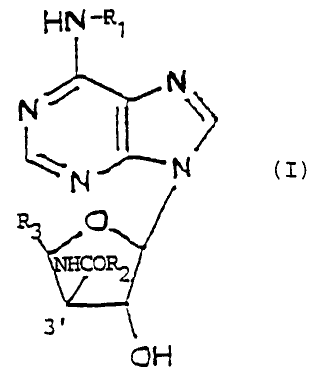
wherein R₁ is a straight or branched alkyl, cycloalkyl, aralkyl or aryl group or H;
R₂ is a straight or branched alkyl, cycloalkyl, aralkyl, aryl group each of which is optionally substituted by at least one C₁₋₄ alkyl, C₁₋₄ alkoxy, nitro group or halogen;
R₃ is CH₂R₄ or CONHR₅;
R₄ is H, OH, OR₅, SR₅;
R₅ is H, an alkyl or a cycloalkyl group;
or a pharmaceutically acceptable salt thereof.

## Description

The invention relates to compounds derived from adenosine and analogs thereof, to the use of such compounds in pharmaceutical compositions as a cardiotonic, a renal protectant, a cognition enhancer and/or an antiasthmatic, to a method and to intermediates for synthesizing such a compound.

Adenosine is a nucleoside which is widely distributed in nature and which has a wide variety of physiological and pharmacological actions.

The physiological and pharmacological actions of adenosine are mediated through specific receptors located on cell surfaces. Three adenosine receptor subtypes are known.

Adenosine receptors are subdivided in A₁, A₂ and the recently identified A₃ receptors, which have been cloned from different species. The A₂ class is further subdivided in A_{2A} and A_{2B}. The effect of numerous modifications of the adenosine scaffold on affinity and intrinsic activity has been investigated by the applicant. In general, substitution at the N⁶ position of the adenine moiety of adenosine, e.g. N⁶-cyclohexyladenosine (CHA) and N⁶-cyclopentyladenosine (CPA), enhances the affinity for the A₁ receptor. Bulky substituents at the 2-position of the adenine moiety, e.g. CGS 21860, increase the A₂ receptor selectively. Ligands for the A₁ and A₂ receptors have also been found in the xanthine series (e.g. 1,3-dipropyl-8-cyclopentylxanthine as antagonist for A₁) and triazoloquinazoline series (e.g. CGS 15943, an antagonist with moderate selectivity for A₂). 2-Chloro-N⁶-(3-iodobenzyl)-5'-methylcarboxamidoadenosine (2Cl-IB-MECA) is an example of an A₃ receptor agonist.

Except for a certain freedom of substitution at the 5'-position, e.g. by a 5'-uronamide moiety, A₁ and A₂ receptors are generally proposed to require an unchanged ribose moiety for adenosine agonist activity. The secondary hydroxyl groups of the ribose moiety are believed to be determinants for the intrinsic activity of adenosine receptor agonists. Most modifications at the 2' and 3' positions of the sugar ring or inversions of chiral centers at these positions were found to abolish adenosine receptor binding. Removal of the 2'- and 3'-hydroxyl groups of N⁶-substituted adenosine derivatives was shown to result in partial agonists, or, in the case of the 2',3'-dideoxy analog of CHA, antagonist properties. Such changes also affect affinity, whereby the drop in A₁ affinity by deletion of the 2'-hydroxyl moiety is more pronounced than the decrease caused by removal of the 3'-group. Removal of the 2'-hydroxyl group of R-PIA (N⁶-(R)-(1-phenyl-2-propyl)adenosine), for example, led to an 800-fold decrease in A₁ receptor affinity, while removal of its 3'-hydroxyl group only led to a 30-fold drop in affinity. The A₁ affinity of 3'-deoxy-R-PIA is reflected in vivo, where significant behavioral (CNS) and cardiovascular effects were observed. Likewise, 3'-deoxy-CPA exhibits submicromolar affinity for the A₁ receptor and can thus be considered as a relatively potent (partial) agonist for this receptor.

In continuation of the synthetic work on ribose-modified adenosine analogs, and in order to further explore the C-3' site for modification, the applicant synthesized a series of 3'-amidated-3'-deoxyxylofuranosyl adenines. Compounds were tested in vitro for their affinity for adenosine receptors, in particular the A₁ receptor. The affinities found in the series of the xylo-analogs prompted the applicant to prepare the compounds according to claim 1.

Surprisingly the 3'-amidated-3'deoxyxylofuranosyladenines, in particular the corresponding N⁶-cyclopentyl derivatives, proved to be well accommodated by the adenosine receptors, in particular the A₁ receptors, with potencies in the lower nanomolar range. Therefore these compounds are considered as potent adenosine receptor antagonists, in particular as A₁ receptor antagonists. This represents a new perspective in the purinergic field. The absence of a GTP-induced shift, i.e., the ratio between the affinities measured in the presence and absence of 1 mM GTP proves the antagonistic behaviour of this new class of CPA analogues.

The compounds according to the invention are described by formula I: 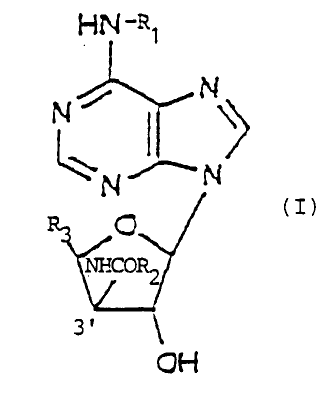
wherein R₁ is a straight or branched alkyl, cycloalkyl, aralkyl or aryl group or H;
R₂ is a straight or branched alkyl, cycloalkyl, aralkyl, aryl group each of which is optionally substituted by at least one C₁₋₄ alkyl, C₁₋₄ alkoxy, nitro group or halogen;
R₃ is CH₂R₄ or CONHR₅;
R₄ is H, OH, OR₅, SR₅;
R₅ is H, an alkyl or a cycloalkyl group;
or a pharmaceutically acceptable salt thereof.

The reaction scheme for the synthesis of a compound according to the invention wherein R₁ is cyclopentyl and R₃ is CH₂OH is explained in detail.

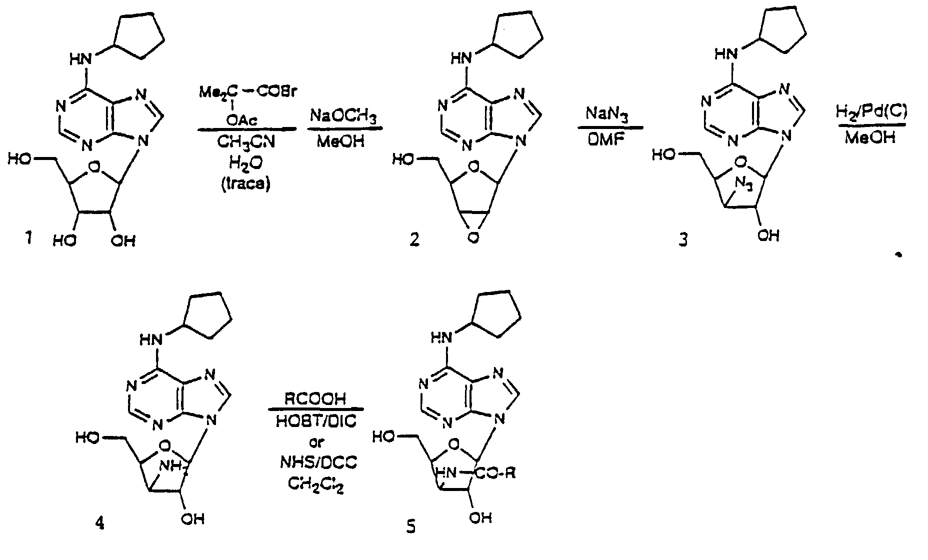

N⁶-Cyclopentyladenosine (1) was converted to its 3'-azidoxylosyl analog using the method of Moffatt and coworkers as it was modified by Hansske and M.J. Robins. Thus, 1 was first converted to its 2',3'-anhydro analog (2) by reaction with α-acetoxyisobutyryl bromide in "moist acetonitrile" followed by alkaline treatment. Epoxide opening of (2) with NaN₃ in hot DMF proceeded stereo- and regioselectively at the 3'-position to give the 3'-azido nucleoside 3 in good yield. Subsequent reduction of 3 to 4 proceeded smoothly by catalytic hydrogenation. Finally, amidation of 4 with suitable carboxylic acids yielded the desired compound according to claim 1 (a-d) wherein R₁ is cyclopentyl and R₃ is CH₂OH. Hereto, the use of N,N'-diisopropylcarbodiimide (DIC) and 1-hydroxybenzotriazole hydrate (HOBT) instead of the DCC-NHS combination was found more convenient with respect to work-up and yield. The general preparation of the compounds according to the present invention is explained below.

To a suspension of 5.71 g (17.03 mmol) N⁶-cyclopentyladenosine **(1)** in 150 mL of dry acetonitrile, containing 15 mL of CH₃CN-H₂O (100:1), was added 9.9 mL (68 mmol) of α-acetoxyisobutyrylbromide and the mixture was stirred at room temperature for 3 h. The clear solution was diluted with 150 mL of saturated aqueous NaHCO₃ solution and extracted twice with 200 mL of EtOAc. The combined organic layers were washed with water, dried (MgSO₄), and evaporated. The residue was dissolved in 0.4 N methanolic sodium methoxide and kept overnight at room temperature. It was then neutralized with acetic acid-water (1:9) and evaporated on Celite. Purification by column chromatography yielded 3.82 g (71% yield) of **(2)** as a white foam.

A mixture of 3.82 g of the epoxide **(2)** and 3.91 g (60 mmol) of NaN₃ in 60 mL of DMF was stirred for 3 days at 80°C. The solvent was evaporated; the residue was adsorbed on Celite and purified by column chromatography (EtOAc, then EtOAc-MeOH, 95:5 and finally 90:10) yielding 2.42 g (42%) of **(3)** as a white solid.

A solution of 2.42 g (6.7 mmol) of **(3)** in 30 mL of MeOH was hydrogenated at room temperature and 1100 psi of pressure in the presence of 10% Pd/C (0.5g) . The mixture was filtered over a Celite pad and evaporated to yield 2.21 g (95%) of **(4)** as a white solid, which was sufficiently pure for the next step.

According to this reaction mechanism the following compounds according to formula I were synthesized.

To a solution of 600 mg (1.72 mmol) of **(4)** in 60 mL of dry CH₂Cl₂-pyridine (5:1) cooled at 0°C, was added 428 mg (1.89 mmol) of benzoic anhydride. After stirring for 3 h at room temperature, the reaction was quenched with 5 mL of H₂O. The mixture was evaporated to dryness and the residue purified by column chromatography (CH₂Cl₂, then CH₂Cl₂-MeOH, 95:5, and finally 90:10) to obtain the crude title compound. Crystallization from acetone and petroleumether gave 500 mg (65%) of pure **(a)**.
a) 9-(3-Benzamido-3-deoxy-β-D-xylofuranosyl)-N⁶-cyclopentyladenine

An amount of 200 mg (0.6 mmol) of **(4)**, 90 mg (0.67 mmol) of HOBT and 0.6 mmol of the respective carboxylic acid were dissolved in 10 mL of CH₂Cl₂. The mixture was cooled at 0°C and 100 µL (0.64 mmol) of DIC was added. After the mixture was stirred for 2 h, it was evaporated and the residue purified on silica gel eluted with CH₂Cl₂-MeOH (98:2 and then 95:5). The desired compounds were obtained as amorphous white powders in 75 to 90% yield. Analytic samples were obtained after crystallization from MeOH **(b-d)**.
b) 9-[3-Deoxy-3-(4-methylbenzamido)-β-D-xylofuranosyl]-N⁶-cyclopentyladenine
c) 9-[3-Deoxy-3-(3,4-dimethylbenzamido)-β-D-xylofuranosyl]-N⁶-cyclopentyladenine
d) 9-[3-Deoxy-3-(3,4-dichlorobenzamido)-β-D-xylofuranosyl]-N⁶-cyclopentyladenine

All modified adenosine and CPA analogs were tested in radioligand binding assays to determine their affinities toward the adenosine A₁ receptor in rat brain cortex (Table 1) . Affinities of the parent 3'-deoxyadenosine and 3'-deoxy-CPA are included for reasons of comparison. The compounds were assayed for A₁ affinity (Ki values expressed in µM) using the tritiated antagonist [³H]-1,3-dipropyl-8-cyclopentylxanthine (DPCPX). Displacement experiments were performed in the absence and presence of 1 mM GTP, allowing the determination of the GTP shift (i.e., the ratio of the apparant Kᵢ values in the presence and absence of GTP, respectively). This shift is an in vitro parameter often indicative for intrinsic activity.

**TABLE 1**

| cmpd | R₁ | 3' substituent | A₁-GTP (µM) | A₁+GTP (µM) | GTP shift |
|---|---|---|---|---|---|
| | H | H | 7.12±3.62 | 15.2±2.0 | 2.1±1.4 |
| | cyclopentyl | H | 0.11±0.03 | 0.47±0.04 | 4.3±1.2 |
| a | cyclopentyl | NHCO-ø | 0.40±0.13 | 0.31±0.05 | 0.80±0.13 |
| b | cyclopentyl | NHCO-ø-pMe | 0.083±0.015 | 0.068±0.010 | 0.84±0.19 |
| c | cyclopentyl | NHCO-ø-3,4-diMe | 0.0236±0.0025 | 0.0174±0.0042 | 0.73±0.11 |
| d | cyclopentyl | NH-CO-ø-3,4-diCl | 0.0325±.00031 | 0.0237±0.0014 | 0.73±0.09 |

Drastic (18-31-fold) improvement in potency towards A₁ receptors was obtained with the N⁶-cyclopentyl congeners, i.e. **a**, suggesting the compatibility of our carbohydrate modification with this typical N⁶-substituent. A series of structural analogs of the benzamido derivative, provides a number of insights into structure-activity relationships with regard to adenosine receptor binding. Varying substituents in para position of the benzamido moiety shows the following trend on A₁ receptor affinity: H < Cl ≤ Me. Adding a further chloro or methyl group in meta on the aromatic ring, to give **c** and **d**, further increases activity. Optimization of the spacer between the phenyl ring and the amido functionality reveals a length of three methylenes to be the most favorable. The 3'-azido intermediate **3** displays a relatively poor affinity for A₁ receptors, suggesting the importance of the amide moiety for effective binding at these receptors. For all analogs synthesized, the GTP shift for the A₁ receptor is nearly 1. The absence of a GTP-induced shift in binding vs radiolabeled DPCPX (1,3-dipropyl-8-cyclopentylxanthine) strongly suggests the 3'-amido xylosyl CPA analogs to be full antagonists for the A₁ receptor. This fact proves that by suitable sugar modification, it is possible to turn a full (CPA) or partial agonist (3'-deoxy-CPA) into an antagonist. As expected, the effect of the N⁶-cyclopentyl substituent is negligible at the A₂ₐ receptors. With the exception of compound 3, the selectivity for A₁ vs A₂ₐ receptors was thus significantly improved (e.g., **c**, the most potent A₁ ligand of this series was found to be 160 times selective vs A₂ₐ).

The crystal structure of compound a reveals the orientation of the ribose group relative to the adenine ring system. The compound appears to be in the so-called syn conformation due to a hydrogen bond formed between N3 and the 5'-OH group (distance N3···H: 2.0 **Å**, angle N3···H-O: 176°). Generally it is assumed that the anti conformation (where the ribose group is turned away from the adenine ring) is essential for agonistic activity. Although certainly not conclusive the syn/anti aspect may explain the antagonistic properties of this new series of compounds.

This invention represents a new perspective in the purinergic field and may provide new non-xanthine leads with improved adenosine receptor antagonist activity.

The invention also relates to a pharmaceutical composition, comprising a compound according to formula (I) as the active ingredient for use as renal protectants, cardiotonics, cognition enhancers and/or antiasthmatics. The composition according to the invention can have the form of powders, suspensions, solutions, sprays, emulsions, infusions, inhalation compositions, unguents or creams and can be used for local application, intranasal, retal, vaginal and also for oral, parenteral (intravenous, intradermal, intramuscular, intrathecal etc.) or transdermal administration, administration by means of inhalation etc. Pharmaceutical compositions of the invention can be prepared by combining (i.e. by mixing, dissolving, etc.) the active compound(s) with pharmaceutically acceptable excipients with neutral character (such as aqueous or non-aqueous solvents, stabilizers, emulsifiers, detergents, additives), and further if necessary colouring agents and flavouring agents. The concentration of the active ingredient in a pharmaceutical composition can vary between 0.001 % and 100 %, depending on the nature of the treatment and the method of administration. The dose of the active ingredient that is administered also depends on the specific application and route of administration, but may for example vary between 0.01 µg and 5 mg per kg body-weight, preferably between 0.1 µg and 500 µg per kg body-weight.

## Claims

1. Compound of the formula (I): 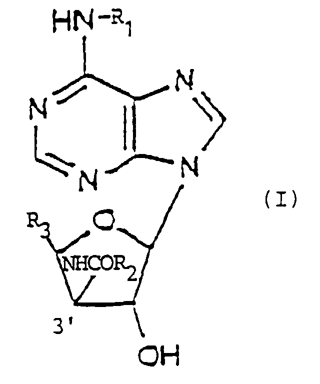
wherein R₁ is a straight or branched alkyl, cycloalkyl, aralkyl or aryl group or H;
R₂ is a straight or branched alkyl, cycloalkyl, aralkyl, aryl group each of which is optionally substituted by at least one C₁₋₄ alkyl, C₁₋₄ alkoxy, nitro group or halogen;
R₃ is CH₂R₄ or CONHR₅;
R₄ is H, OH, OR₅, SR₅;
R₅ is H, an alkyl or a cycloalkyl group;
or a pharmaceutically acceptable salt thereof.

2. Compound according to claim 1, wherein R₂ is alkylphenyl or phenyl each of which is optionally substituted by at least one C₁₋₄ alkyl, C₁₋₄ alkoxy, nitro group or halogen.

3. Compound according to claim 2, wherein R₂ is a phenyl group optionally substituted by CH₃, OCH₃, NO₂ or halogen.

4. Compound according to claim 3, wherein R₂ is a phenyl group optionally substituted at the meta and/or para position by CH₃, OCH₃, NO₂ and/or halogen.

5. Compound according to claim 3, wherein R₂ is a phenyl group in which para- and meta position substituted by CH₃ or halogen.

6. Compound according to one of the claims 1-5, wherein R₁ is a C₃₋₁₀ cycloalkyl group and preferably a C₄₋₈ cycloalkyl group, and more preferably a C₅₋₆ cycloalkyl group.

7. Compound according to claim 6, wherein R₁ is cyclopentyl.

8. Compound according to one of the claims 1-7, wherein R₃ is CH₂OH.

9. Compound according to claim 1, which is 9-[3-Deoxy-3-(4-methylbenzamido)-β-D-xylofuranosyl]-N⁶-cyclopentyladenine.

10. Compound according to claim 1, which is 9-[3-Deoxy-3-(3 4-dimethylbenzamido)-β-D-xylofuranosyl]-N⁶-cyclopentyladenine.

11. Compound according to claim 1, which is 9-[3-Deoxy-3-(3,4-dichlorobenzamido)-β-D-xylofuranosyl]-N⁶-cyclopentyladenine.

12. Compound according to one of the claims 1-11 for use as a cardiotonic, a renal protectant, a cognition enhancer and/or an antiasthmatic.

13. Composition comprising a pharmaceutically effective amount of a compound according to one of the claims 1-12 and a pharmaceutically acceptable carrier thereof.

14. Method for treating a patient in need of a cardiotonic, a renal protectant, a cognition enhancer and/or an antiasthmatic comprising administering to said patient an effective amount of a compound according to one of the claims 1-13 and a pharmaceutically acceptable carrier thereof.

15. Method for the preparation of a compound according to one of the claims 1-12, wherein a compound of the formula (II): 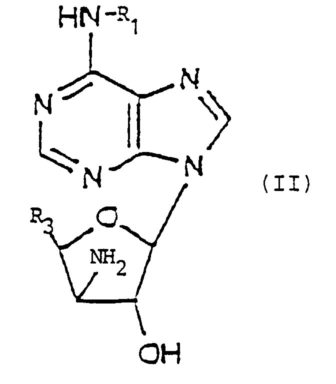 is treated with R₂COOH in the presence of N,N'-diisopropylcarbodiimide (DIC)/1-hydroxybenzotriazole hydrate (HOBT),
wherein R₁ is a straight or branched alkyl, cycloalkyl, aralkyl or aryl group or H;
R₂ is a straight or branched alkyl, cycloalkyl, aralkyl, aryl group each of which is optionally substituted by at least one C₁₋₄ alkyl, C₁₋₄ alkoxy, nitro group or halogen;
R₃ is CH₂R₄ or CONHR₅;
R₄ is H, OH, OR₅, SR₅;
R₅ is H, an alkyl or a cycloalkyl group.

16. Method for the preparation of a compound of the formula (II), wherein a compound of the formula (III): 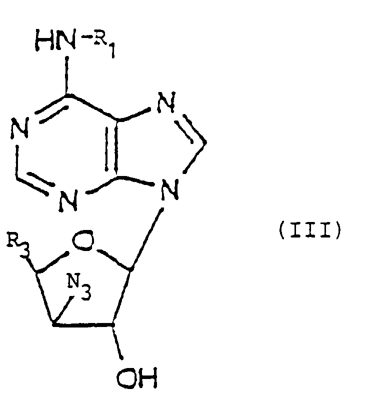 is catalytically hydrogenated,
wherein R₁ is a straight or branched alkyl, cycloalkyl, aralkyl or aryl group or H;
R₃ is CH₂R₄ or CONHR₅;
R₄ is H, OH, OR₅, SR₅;
R₅ is H, an alkyl or a cycloalkyl group.

17. Method for the preparation of a compound of the formula (III), wherein a compound of the formula (IV): 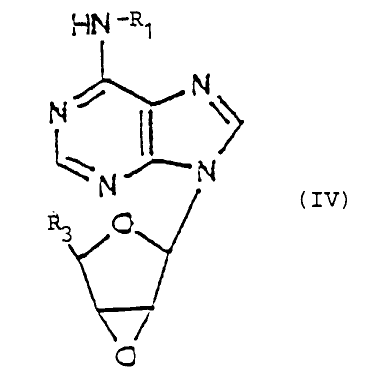 is treated with NaN₃ in hot DMF,
wherein R₁ is a straight or branched alkyl, cycloalkyl, aralkyl or aryl group or H;
R₃ is CH₂R₄ or CONHR₅;
R₄ is H, OH, OR₅, SR₅;
R₅ is H, an alkyl or a cycloalkyl group.

18. Compound having the formula: 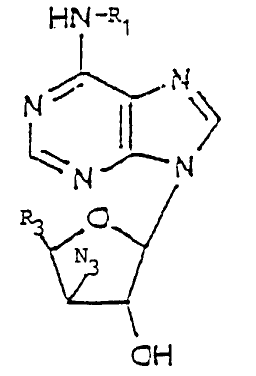
wherein R₁ is a straight or branched alkyl, cycloalkyl, aralkyl or aryl group or H;
R₃ is CH₂R₄ or CONHR₅;
R₄ is H, OH, OR₅, SR₅;
R₅ is H, an alkyl or a cycloalkyl group.

19. Compound having the formula:
